(19) Europäisches Patentamt
European Patent Office
Office européen des brevets

(11) EP 4 458 890 A1

(12) **EUROPEAN PATENT APPLICATION**
published in accordance with Art. 153(4) EPC

(43) Date of publication:
**06.11.2024 Bulletin 2024/45**

(21) Application number: **22916711.9**

(22) Date of filing: **27.12.2022**

(51) International Patent Classification (IPC):
***C08J 7/06*** *(2006.01)* ***A61L 27/34*** *(2006.01)*

(52) Cooperative Patent Classification (CPC):
**A61L 27/34; C08J 7/06**

(86) International application number:
**PCT/KR2022/021424**

(87) International publication number:
**WO 2023/128570 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.12.2021 KR 20210192254**

(71) Applicants:
- **i-Sens, Inc.**
  **Seoul 06646 (KR)**
- **Ulsan National Institute of Science and Technology**
  **Ulju-gun, Ulsan 44919 (KR)**

(72) Inventors:
- **SHIN, Hyun Seo**
  **Seoul 06646 (KR)**
- **CHO, Joo Yong**
  **Seoul 06646 (KR)**
- **KIM, Su Jin**
  **Seoul 06646 (KR)**
- **YANG, Bo Na**
  **Seoul 06646 (KR)**
- **KANG, Geun Hee**
  **Seoul 06646 (KR)**
- **RYU, Ja Hyoung**
  **Ulju-gun, Ulsan 44919 (KR)**
- **KIM, Do Hyun**
  **Ulju-gun, Ulsan 44919 (KR)**
- **PARK, Ga Eun**
  **Ulju-gun, Ulsan 44919 (KR)**
- **YANG, Gyeong Seok**
  **Ulju-gun, Ulsan 44919 (KR)**
- **CHOI, Eun Seong**
  **Ulju-gun, Ulsan 44919 (KR)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(54) **COMPOSITION, KIT AND METHOD FOR SURFACE TREATMENT OF POLYMER MATERIAL**

(57) The present invention relates to a composition for the surface treatment of a polymer material, and more specifically, to a composition which, by comprising glycidol-derived repeating units, and a hyperbranched polyglycerol containing an unsubstituted or substituted acrylate group at at least one end thereof, has the effect of suppressing fibrosis, thrombus formation, or inflammatory cell adsorption by preventing proteins from being adsorbed to the surface of a polymer material that is surface-treated with the composition or an in-vivo implantation material including a polymer material.

EP 4 458 890 A1

[FIG. 1]

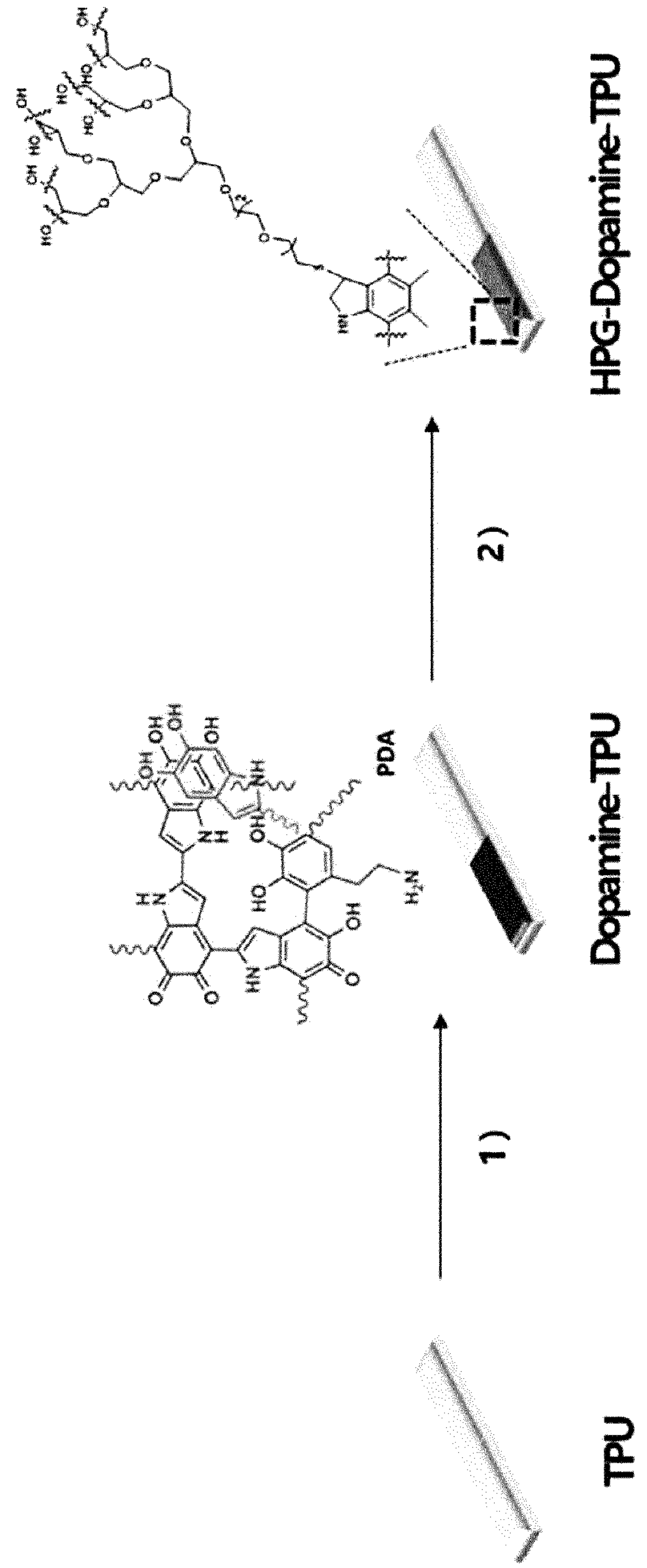

## Description

[Technical Field]

The present invention relates to a composition for surface treatment of a polymer material, a kit and a method for surface treatment of a polymer material.

[Background Art]

Recently, due to growth of aging populations in modern society and development of medical devices, interest in and demand for artificial implants which serve to diagnose diseases or assist functions of body tissues/organs are rapidly increasing. Biological materials forming the implant include various types of materials such as metals, ceramics, and polymers, etc., and are being diversely applied to in-vivo implantable devices such as medical devices including a catheter and a stent which directly come into contact with the blood, biosensors, drug delivery systems and the like. The biological materials forming the artificial implants should be made of inert substances which do not affect activities within the body at all, and should be made of materials with excellent biocompatibility because they are in direct contact with the tissues and blood in the body for a long period of time. To this end, researches on surface treatment technologies for the biological materials are continuously being carried out.

Meanwhile, artificial biological materials inserted into the body from an outside are recognized as foreign substances by the body immune system, thereby causing a foreign body reaction. Starting with the adsorption of proteins within the tissues and blood to the surfaces of the materials, platelets or macrophages, etc. gather around them while the adsorbed proteins are denatured. Since most of the implants are too large to be swallowed by phagocytosis, the gathered macrophages are fused to form multinucleated giant cells (foreign body giant cells), and signaling factors generated from the same cause fibroblasts to gather around them to form a fibrous capsule membrane made of collagen.

Biofouling refers to that the proteins are absorbed to the surfaces of the biological materials. This protein adsorption is a phenomenon of initiating a foreign body reaction, and subsequently promotes the fibrosis, thrombus formation, and inflammatory cell adsorption, thereby inducing inflammation. Therefore, a surface treatment technology for changing the surface of the material to one having a property that can inhibit the adsorption of proteins is receiving attention as a way to minimize the foreign body reaction, which causes a problem when inserting the artificial biological materials into the body.

Anti-fouling coating refers to coating a surface with the biocompatible material to prevent biological substances such as proteins, cells and microorganisms from being adhered to the surface. In the case of existing artificial implants, even medical silicone and Gore-Tex, which are known to be highly biocompatible, often cause the foreign body reaction and were limited in application thereof by side effects of causing inflammation and pain, etc., due to the thrombus formation or hypertrophy of fibrotic tissue. Many researchers feel the need to develop a surface of an anti-foreign body reactive material, and a method for surface treatment using a polymer with anti-fouling properties is receiving attention. This improves biocompatibility problems that cause the foreign body reaction and can be applied to an implantable medical device or biosensor for disease diagnosis, thus the applicable scope thereof is limitless.

[Summary of Invention]

[Problems to be Solved by Invention]

An object of the present invention is to provide a composition for surface treatment of a polymer material.

Another object of the present invention is to provide a kit for surface treatment of a polymer material.

In addition, another object of the present invention is to provide an in-vivo implantation material including a surface-treated polymer material.

Further, another object of the present invention is to provide a method for surface treatment of a polymer material.

[Means for Solving Problems]

1. A composition for surface treatment of a polymer material comprising a hyperbranched polyglycerol comprising a glycidol-derived repeating unit and an unsubstituted or substituted acrylate group at least at one end thereof.
2. The composition for surface treatment of a polymer material according to the above 1, wherein the hyperbranched polyglycerol has a number average molecular weight of 500 to 500,000 g/mol.
3. The composition for surface treatment of a polymer material according to the above 1, wherein the hyperbranched

polyglycerol is subjected to ring-opening polymerization of glycidol using tritylated triethylene glycol as an initiator.

4. The composition for surface treatment of a polymer material according to the above 1, wherein the substituted acrylate is an acrylate in which carbon No. 2 is substituted with C1 to C6 straight or branched chain alkyl.

5. The composition for surface treatment of a polymer material according to the above 1, wherein the hyperbranched polyglycerol is a hyperbranched polyglycerol in which at least some of its ends are methacrylated.

6. The composition for surface treatment of a polymer material according to the above 5, wherein the methacrylated hyperbranched polyglycerol is prepared by reacting the hyperbranched polyglycerol with glycidyl methacrylate.

7. The composition for surface treatment of a polymer material according to the above 1, wherein the polymer material includes at least one selected from the group consisting of thermoplastic polyurethane (TPU), polycarbonate (PC), polydimethylsiloxane (PDMS), polyvinyl chloride (PVC), polyethylene glycol (PEG), silicone, Teflon (PTFE), polystyrene, nylon, polyethylene terephthalate (PET), polyacrylate, polypropylene (PP), polyethylene (PE), polyetheretherketone (PEEK), polysulfone (PS), phenol resin, epoxy resin, polyglycolide (PGA), polylactide (PLLA), polycaprolactone (PCL), poly(lactic-co-glycolic acid) (PLGA), poly(ε-caprolactone-co-lactide) (PLCL), polydioxanone (PDO), polytrimethylene carbonate (PTMC), polyanhydride, polyorthoester, polyphosphagen, hyaluronic acid, alginic acid, chitosan, collagen, gelatin and polyamino acid.

8. A kit for surface treatment of a polymer material including: the composition according to any one of the above 1 to 7; and a crosslinking agent solution.

9. The kit for surface treatment of a polymer material according to the above 8, wherein the crosslinking agent solution is prepared by dissolving a crosslinking agent in C1 to C6 alcohol, C3 to C6 cycloalkanone, or C3 to C6 oxacycloalkane.

10. The kit for surface treatment of a polymer material according to the above 8, wherein the crosslinking agent is PETMP(pentaerythritol tetra(3-mercaptopropionate), pentaerythritol tetrakis(3-mercaptopropionate)).

11. An in-vivo implantation material including a polymer material subjected to surface treatment with the composition according to any one of the above 1 to 7.

12. The in-vivo implantation material according to the above 11, wherein the in-vivo implantation material includes a continuous blood glucose monitoring sensor.

13. A method for surface treatment of a polymer material including treating at least a portion of a surface of the polymer material with the composition according to any one of the above 1 to 6 and a crosslinking agent.

14. The method for surface treatment of a polymer material according to the above 13, wherein the step of treating is performed by immersing the polymer material in the composition, and then immersing in the crosslinking agent.

15. The method for surface treatment of a polymer material according to the above 13, wherein the crosslinking agent is PETMP(pentaerythritol tetra(3-mercaptopropionate), pentaerythritol tetrakis(3-mercaptopropionate)).

16. The method for surface treatment of a polymer material according to the above 13, wherein the crosslinking agent is dissolved in C1 to C6 alcohol, C3 to C6 cycloalkanone, or C3 to C6 oxacycloalkane.

17. The method for surface treatment of a polymer material according to the above 13, wherein a concentration of the crosslinking agent is 10 mM to 30 mM.

18. The method for surface treatment of a polymer material according to the above 13, wherein the polymer material includes at least one selected from the group consisting of thermoplastic polyurethane (TPU), polycarbonate (PC), polydimethylsiloxane (PDMS), polyvinyl chloride (PVC), polyethylene glycol (PEG), silicone, Teflon (PTFE), polystyrene, nylon, polyethylene terephthalate (PET), polyacrylate, polypropylene (PP), polyethylene (PE), polyetheretherketone (PEEK), polysulfone (PS), phenol resin, epoxy resin, polyglycolide (PGA), polylactide (PLLA), polycaprolactone (PCL), poly(lactic-co-glycolic acid) (PLGA), poly(ε-caprolactone-co-lactide) (PLCL), polydioxanone (PDO), polytrimethylene carbonate (PTMC), polyanhydride, polyorthoester, polyphosphagen, hyaluronic acid, alginic acid, chitosan, collagen, gelatin and polyamino acid.

[Advantageous effects]

The in-vivo implantation material including a polymer material subjected to surface treatment with the composition of the present invention may suppress in-vivo protein adsorption, surface fibrosis, and inflammatory cell adsorption on the surface of the implantation material, and may prevent a deterioration in performance thereof even when ultimately inserting it into the body.

In addition, the in-vivo implantation material including a polymer material subjected to surface treatment with the composition of the present invention may delay the thrombus formation or inflammatory response in the body, which will occur when inserting it into the body.

[Brief Description of Drawings]

FIG. 1 schematically illustrates a process of performing treatment on the surface of a polymer material using hyperbranched polyglycerol having a glycidol-derived repeating unit and containing dopamine.
FIG. 2 illustrates a process of synthesizing hyperbranched polyglycerol from triethylene glycol.
FIG. 3 illustrates $^1$H NMR analysis results of H2 shown in FIG. 2.
FIG. 4 illustrates $^1$H NMR analysis results of H3 shown in FIG. 2.
FIG. 5 illustrates $^1$H NMR analysis results of H4 shown in FIG. 2.
FIG. 6 illustrates a process of synthesizing the methacrylated hyperbranched polyglycerol from H4 shown in FIG. 2.
FIG. 7 illustrates $^1$H NMR analysis results of the methacrylated hyperbranched polyglycerol.
FIG. 8 illustrates a process of coating the surface of the polymer material with the hyperbranched polyglycerol and dopamine under basic buffer solvent conditions and results thereof.
FIG. 9 illustrates a process of coating the surface of a polymer material with the hyperbranched polyglycerol and dopamine under methanol solvent conditions and results thereof.
FIGS. 10 and 11 illustrate a process of coating the surface of the polymer material with the methacrylated hyperbranched polyglycerol and PETMP reducing agent under methanol (FIG. 10), ethanol (FIG. 11), and cyclopentanone (FIG. 11) solvent conditions and results thereof.

[Mode for Carrying out Invention]

Hereinafter, the present invention will be described in detail.
The present invention provides a composition for surface treatment of a polymer material.
The composition for surface treatment of a polymer material includes hyperbranched polyglycerol having a glycidol-derived repeating unit.
The hyperbranched polyglycerol is a polymer having a branched structure, and includes a plurality of hydroxyl groups.
The hyperbranched polyglycerol may have a number average molecular weight (Mn) of 500 to 500,000 g/mol, 650 to 400,000 g/mol, 800 to 300,000 g/mol, 950 to 200,000 g/ mol, 1,100 to 100,000 g/mol, 1,400 to 50,000 g/mol, 2,000 to 25,000 g/mol, 2,500 to 12,000 g/mol, or 3,000 to 6,000 g/mol, but it is not limited thereto. For example, the number average molecular weight may be a value calculated using the number of protons by measuring NMR.
A degree of branching (DB) of the hyperbranched polyglycerol may be 0.4 to 1, 0.5 to 0.9, or 0.6 to 0.8, but it is not limited thereto.
The hyperbranched polyglycerol may further include a polyethylene glycol group at one end thereof. As an example, the hyperbranched polyglycerol may have a structure in which triethylene glycol is bound to one end thereof.
The hyperbranched polyglycerol may be prepared by methods known to those skilled in the art.
The hyperbranched polyglycerol may be subjected to ring-opening polymerization of glycidol using triethylene glycol to which an acid-decomposable protective group or a photodegradable protective group is attached as an initiator. As used herein, the protective group refers to a group to which a specific functional group is bound so as to prevent it from participating in a reaction, and an acid-decomposable protective group or photodegradable protective group refers to a protective group in which the functional group bound thereto can be deprotected by decomposing with acid or light.
The acid-decomposable or photodegradable protective group may be a tosyl group, O-trityl, N-trityl or S-trityl group, but it is not limited thereto.
The triethylene glycol to which an acid-decomposable protective group or a photodegradable protective group is attached may be tritylated triethylene glycol, and specifically, may be S-tritylated triethylene glycol.
The hyperbranched polyglycerol may be a hyperbranched polyglycerol having a glycidol-derived repeating unit and including an unsubstituted or substituted acrylate group at least at one end thereof.
The unsubstituted acrylate group may be represented by Formula 1 below.

[Formula 1]

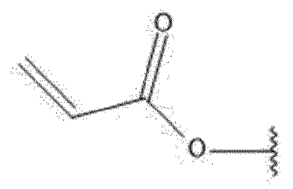

The substituted acrylate group may be an acrylate group in which carbon No. 2 of Formula 1 above is substituted

with C1 to C6 straight or branched chain alkyl. For example, the substituted acrylate group may be a methacrylate group.

According to one embodiment, the hyperbranched polyglycerol may be acrylated hyperbranched polyglycerol. Specifically, the hyperbranched polyglycerol may be a hyperbranched polyglycerol in which at least some of its ends are acrylated.

According to one embodiment, the hyperbranched polyglycerol may be methacrylated hyperbranched polyglycerol. Specifically, the hyperbranched polyglycerol may be a hyperbranched polyglycerol in which at least some of its ends are methacrylated. The methacrylated hyperbranched polyglycerol may be prepared by the methods known in the art, and for example, may be prepared by reacting the hyperbranched polyglycerol with glycidyl methacrylate.

The polymer material may be used as a biological material. The biological material is collectively called materials that can be applied to the living body as a means for diagnosing, treating and preventing diseases, and means basic materials such as artificial organs, artificial tissues and therapeutic products, which are used to replace damaged or dysfunctional human tissues and organs.

The polymer material may include at least one selected from the group consisting of thermoplastic polyurethane (TPU), polycarbonate (PC), polydimethylsiloxane (PDMS), polyvinyl chloride (PVC), polyethylene glycol (PEG), silicone, Teflon (PTFE), polystyrene, nylon, polyethylene terephthalate (PET), polyacrylate, polypropylene (PP), polyethylene (PE), polyetheretherketone (PEEK), polysulfone (PS), phenol resin, epoxy resin, polyglycolide (PGA), polylactide (PLLA), polycaprolactone (PCL), poly(lactic-co-glycolic acid) (PLGA), poly(ε-caprolactone-co-lactide) (PLCL), polydioxanone (PDO), polytrimethylene carbonate (PTMC), polyanhydride, polyorthoester, polyphosphagen, hyaluronic acid, alginic acid, chitosan, collagen, gelatin and polyamino acid, but it is not limited thereto.

The term "surface treatment" used herein means modifying a surface of a treating target to change properties of the surface, the treatment includes coating the surface, and may be intended to minimize physical, chemical or biological phenomena and reactions which may occur on the surface of the treating target. Specifically, when the treating target is a polymer material, the surface treatment may be intended to minimize the inflammatory cell adsorption or inflammatory response.

The composition may be used, for example, after a dopamine solution is first applied to the surface of the polymer material. In this case, a coating film may be formed on the surface of the polymer material through a thiol-ene click chemical reaction between the dopamine and the hyperbranched polyglycerol applied to the surface of the polymer material.

The composition may be used, for example, with a crosslinking agent. In this case, crosslinks may be formed between the surface of the polymer material and the hyperbranched polyglycerol, thereby performing surface treatment of the polymer material in a more stable state.

The hyperbranched polyglycerol included in the composition of the present invention includes a branch structure to exhibit a high density, such that it may show effects of preventing proteins from being adhered to the surface of the polymer material and suppressing fibrosis through steric hindrance. In addition, the hyperbranched polyglycerol includes a plurality of hydroxyl groups, thereby showing high hydrophilicity and exhibiting excellent biocompatibility. Considering these characteristics, the polymer material subjected to surface treatment with the composition of the present invention may be used as a component of an in-vivo implantation material.

When performing treatment on the surface of the polymer material using the composition of the present invention and dopamine or a crosslinking agent, in-vivo inflammatory response may be delayed even if the surface-treated polymer material is implanted into the body.

In addition, the present invention provides a kit for surface treatment of a polymer material.

The kit for surface treatment of a polymer material may include the composition for surface treatment of a polymer material including the hyperbranched polyglycerol.

The kit may further include a dopamine solution. The dopamine solution may be prepared, for example, by dissolving dopamine in a buffer solution under basic conditions or methanol, and specifically, may be prepared by dissolving dopamine in methanol, but it is not limited thereto. When the kit further includes the dopamine solution, it may additionally include an oxidant. The oxidant may include, for example, $NaIO_4$, but it is not limited thereto.

The hyperbranched polyglycerol may be a hyperbranched polyglycerol having a glycidol-derived repeating unit and including an unsubstituted or substituted acrylate group at at least one end thereof. In this case, the kit may further include a crosslinking agent solution. The crosslinking agent solution may be prepared, for example, by dissolving a crosslinking agent in C1 to C6 alcohol (e.g., methanol, ethanol), C3 to C6 cycloalkanone (e.g., cyclopentanone) or C3 to C6 oxacycloalkane (e.g., tetrahydrofuran), specifically may be prepared by dissolving a crosslinking agent in cyclopentanone or tetrahydrofuran, and more specifically, may be prepared by dissolving a crosslinking agent in cyclopentanone, but it is not limited thereto. The crosslinking agent may be used without limitation as long as it can crosslink the polymer material and the hyperbranched polyglycerol, and may include, for example, PETMP(pentaerythritol tetra(3-mercaptopropionate), pentaerythritol tetrakis(3-mercaptopropionate)), but it is not limited thereto. The PETMP contains four thiol groups, and forms crosslinks between methacryl groups of the methacrylated hyperbranched polyglycerol and the surface of the polymer material under conditions of irradiation with light or heating at an appropriate temperature, thereby allowing

the composition of the present invention to be more stably treated on the surface of the polymer material.

In addition, the present invention provides an in-vivo implantation material.

The in-vivo implantation material includes the polymer material subjected to surface treatment with the composition for surface treatment of a polymer material.

The in-vivo implantation material is a material that can be inserted or implanted into the body, and includes materials that can exist temporarily as well as materials that can exist permanently or semi-permanently in the body.

The surface-treated polymer material included in the in-vivo implantation material may be disposed at the outermost region of the implantation material. In this case, the proteins adsorbed on the implantation material are reduced by the surface-treated polymer material disposed at the outermost region, thereby suppressing the fibrosis, thrombus formation or inflammatory cell adsorption in the body.

The in-vivo implantation material may include at least one selected from the group consisting of a biosensor, artificial blood vessel, catheter, drain, shunt, cannula, tube, guide wire, bone chip, conduit, pin, rod, screw, plate, suture, patch, balloon, stent, membrane, dental implant, and dental material, a support for tissue regeneration, a drug delivery system and a gene delivery system, specifically may include the biosensor, and more specifically, may include a continuous blood glucose monitoring sensor, but it is not limited thereto.

Further, the present invention provides a method for surface treatment of a polymer material.

As an example, the method for surface treatment of a polymer material may include treating a dopamine-coated polymer material with the composition for surface treatment of a polymer material including the hyperbranched polyglycerol.

The polymer material, surface treatment, and composition may be within the above-described range, but they are not limited thereto.

The dopamine-coated polymer material may be prepared by the methods known to those skilled in the art, and may be dip-coated, for example, by immersing the polymer material in a dopamine solution, but it is not limited thereto.

As another example, the method for surface treatment of a polymer material may include the step of treating at least a portion of the surface of the polymer material with a composition for surface treatment of a polymer material including hyperbranched polyglycerol containing an unsubstituted or substituted acrylate group at at least one end thereof and a crosslinking agent.

The substituted acrylate group may be an acrylate group in which carbon No. 2 of the acrylate is substituted, and specifically, may be an acrylate group in which carbon No. 2 of the acrylate is substituted with C1 to C6 straight or branched chain alkyl. According to one embodiment, the substituted acrylate may be methacrylate.

According to one embodiment, the method for surface treatment of a polymer material may include the step of treating at least a portion of the surface of the polymer material with a composition for surface treatment of a polymer material including methacrylated hyperbranched polyglycerol and a crosslinking agent.

The step of treating with the composition for surface treatment of a polymer material including hyperbranched polyglycerol containing an unsubstituted or substituted acrylate group at at least one end thereof and a crosslinking agent may be performed by the methods known to those skilled in the art, and may be performed, for example, by immersing the polymer material in the composition and then immersing in the crosslinking agent, but it is not limited thereto.

The crosslinking agent may be used without limitation as long as it can crosslink the polymer material and the hyperbranched polyglycerol, and may include, for example, PETMP(pentaerythritol tetra(3-mercaptopropionate), pentaerythritol tetrakis(3-mercaptopropionate)), but it is not limited thereto. The crosslinking agent may be dissolved, for example, in C1 to C6 alcohol (e.g., methanol, ethanol), C3 to C6 cycloalkanone (e.g., cyclopentanone) or C3 to C6 oxacycloalkane (e.g., tetrahydrofuran), specifically may dissolved in cyclopentanone or tetrahydrofuran, and more specifically, may be dissolved in cyclopentanone, but it is not limited thereto. A concentration of the crosslinking agent may be, for example, 10 to 30 mM, 12 to 29 mM, 15 to 28 mM, 16 to 26 mM, 18 to 25 mM, 18.5 to 24.5 mM, 19 to 24 mM or 19.5 to 23.5 mM, but it is not limited thereto.

The polymer material to be the target of the method for surface treatment of a polymer material may be included in the in-vivo implantation material, and specifically, the polymer material may be disposed at the outermost region of the implantation material. The in-vivo implantation material may be within the above-described range, but it is not limited thereto.

When using the polymer material surface-treated by the method of the present invention or an in-vivo implantation material including the polymer material, there are effects of preventing the proteins from being adsorbed to the surface, thereby suppressing the fibrosis, thrombus formation, or inflammatory cell adsorption in the body.

Hereinafter, the present invention will be described in detail with reference to the following examples.

## Example

The present inventors have confirmed that, by using hyperbranched polyglycerol (HPG), which is polyol having numerous hydroxyl groups (-OH) present therein and has a tree branch shape and dopamine (FIG. 1), it was possible

to utilize them as an anti-fouling coating material. The HPG exhibits a high density thus to prevent the adsorption of proteins through steric hindrance, and shows high hydrophilicity, such that it is expected to be biocompatible.

## Analysis equipment and conditions

$^1H$ NMR spectrum was measured using a nuclear magnetic resonance spectrometer (Agilent 400MHz FT-NMR). Molecular weight and polydispersity index (PDI) values were measured using gel permeation chromatography (GPC, Agilent 1260 infinity) in aqueous buffer containing 0.05 M sodium nitrate ($NaNO_3$). To this end, a Shodex SB-803HQ column was used at 35°C, and a flow rate was set at 1.0 ml/min.

Hydrophilicity of a substrate was evaluated using a water contact angle meter (Phoenix 300).

Surface morphology and roughness of the substrate were measured using an atomic force microscope (Multimode V_AFM, Veeco). Samples were measured using tapping mode in air conditions. The chemical composition of the substrate surface was analyzed using X-ray photoelectron spectroscopy (XPS, K-alpha, ThermoFisher). During preparing samples for thickness analysis, a cross-section of the substrate was cut using a microtome (Ultramicrotome, CR-X, RMC). To measure the surface morphology and thickness, images of an upper portion and cross section of the substrate were captured at 14 kV using a cold scanning electron microscope (Cold FE-SEM, S-4800, Hitachi High-Technologies) after performing coating treatment with metal.

## Step 1. Synthesis and analysis of biocompatible polymer

### 1-1. Hyperbranched polyglycerol (HPG)

After performing tosylation (H2) using triethylene glycol, trityl modification conjugation (H3) was performed. Using the trityl-modified triethylene glycol as an initiator, ring-opening polymerization (H4) of the glycide monomer was performed on a hydroxyl group (FIG. 2).

Specifically, a method for synthesizing H2, H3 and H4 shown in FIG. 2 is as follows.

To synthesize H2, 4-toluenesulfonyl chloride (1.1 g, 1.0 eq.) and 10 ml of dichloromethane were put into a single-neck round-bottom flask, cooled to 0 °C, and then triethylene glycol (1.0 g, 1.2 eq.) and triethyl amine (0.79 g, 1.3 eq.) were added thereto, followed by stirring for 2 hours. Thereafter, non-reactive gas was created using argon at room temperature, followed by stirring for 24 hours. After completion of the reaction, the produced solid was removed by filtration under reduced pressure. The filtrate was concentrated through a reduced pressure concentration evaporator and separated using silica gel chromatography under 20% hexane and 80% ethyl acetate conditions ($^1H$ NMR (400 MHz, $CDCl_3$): $\delta$ 7.81-7.79 (d, 2H), 7.35-7.33 (d, 2H), 4.18-4.15 (t, J = 4.8 Hz, 2H), 3.73-3.59 (m, 11H), 2.45 (s, 3H), FIG. 3).

To synthesize H3, a solution of sodium hydroxide (74 mg, 1.25 eq.) dissolved in 1.0 ml of distilled water was put into a solution of triphenylmethanethiol (0.51 g, 1.25 eq.) dissolved in an ethanol/toluene (1:1, 5.0 ml) solvent, followed by stirring. Thereafter, H2 (1.0 g, 1.2 eq.) was dissolved in the ethanol/toluene (1:1, 5.0 ml) solvent, and added to the previously stirred solution. After performing a reaction for 18 hours at room temperature, the reactant was poured into a saturated sodium bicarbonate solution prepared in advance. An organic solution layer was washed through extraction of 3 times with sodium bicarbonate and 3 times with salt water. Thereafter, the reactant was dried with magnesium sulfate, concentrated using the reduced pressure concentration evaporator under high vacuum, and separated while increasing a ratio of ethyl acetate from 50% hexane and 50% ethyl acetate conditions to 25% hexane and 75% ethyl acetate conditions using silica gel chromatography ($^1H$ NMR (400 MHz, DMSO): $\delta$: 7.43-7.25 (m 12H), 7.42-7.40 (m, 3H), 3.72-3.68 (m, 2H), 3.60-3.55 (m, 4H), 3.47-3.43 (m, 2H), 2.46-2.42 (t, 2H), FIG. 4)

To synthesize H4, H3 (0.1 g, 1.0 eq.) and sodium hydride (68 mg, 0.1 eq.) were dried under high vacuum for 10 minutes, then the flask gas was replaced with nitrogen, and glycidyl monomer (1.903 ml, 0.1 eq.) was added thereto at 95 °C for 20 hours using a syringe pump. After injection was completed, an additional reaction was performed for 4 hours to allow all remaining glycidyl groups to react, followed by cooling to room temperature. The reactant was dissolved in a minimum amount of methanol, then precipitated in diethyl ether, and the solid was separated using a centrifuge. After repeating the precipitation 3 times, the obtained product was dried ($^1H$ NMR (400 MHz, DMSO): $\delta$: 7.43-7.25 (m 12H), 7.42-7.40 (m, 3H), 4.79-4.42 (m, 114H), 3.75-3.27 (m, 614H), FIG. 5).

Through $^1H$ NMR analysis of H4, alkyl chains of the hydroxyl group (4.79 ppm - 4.42 ppm) and polyether group (3.75 ppm - 3.27 ppm) were confirmed. NMR was measured to determine the molecular weight of the polymer using calculations through the number of protons. The degree of polymerization (DP) was calculated as "a value obtained by subtracting 1 from [an integrated value of $^1H$ proton NMR signal corresponding to the hydroxyl group / an integrated value of $^1H$ proton NMR signal corresponding to the trityl initiator]" (FIG. 5).

Therefore, the molecular weight of the polymer was calculated using an equation:

Molecular weight of polymer = [Molecular weight before polymerization + Degree of polymerization * Molecular weight

of monomer repeat unit].

$$(M_n = M_I + DP \cdot M_{glycidol} = M_I + [I_{OH}/I_{Trityl} - 1] \cdot M_{glycidol};$$

$M_n$: molecular weight of the polymer,
$M_I$ molecular weight of polymer initiator,
$I_{OH}$: integrated value of $^1H$ proton NMR signal corresponding to the hydroxyl group,
$I_{Trityl}$: integrated value of a signal of $^1H$ proton NMR corresponding to the trityl initiator,
$M_{glycidol}$: molecular weight of monomer repeat unit)

The integrated value of NMR signal corresponding to the hydroxyl group (4.79 ppm - 4.42 ppm) is 124.40, and it has 15 protons per unit of trityl initiator. Therefore, the molecular weight of "350.48 + [((124.40/15) - 1) * 74.079] = 890.76" was confirmed using the corresponding calculation equation.

1-2. Methacrylated hyperbranched polyglycerol

The H4 polymer obtained by the above-described method was reacted with glycidyl methacrylate to modify and conjugate the H4 polymer to glycidyl methacrylate. Thereafter, a crosslinking reaction was performed with a reducing agent having a thiol group, then the substrate was coated with the prepared product (FIG. 6).

Through NMR analysis after the modification and conjugation, a double bond signal of the alkyl group of methacrylic acid was found around 5.5 ppm - 6.0 ppm, and a signal attenuation of the hydroxyl group around 4.0 ppm - 5.0 ppm was observed due to the modification and conjugation (FIG. 7).

**Step 2. Evaluation of polymer fixation ability on polyurethane substrate - stability evaluation**

To coat a thermoplastic polyurethane (TPU) substrate with HPG, coating was performed using the following three methods.

2-1. Oxidation polymerization coating

In the field of dopamine coating, a continuous coating strategy was adopted to induce a formation of polydopamine through oxidative polymerization using 1.0 M basic buffer (pH 8.5), and then HPG was coated on the same through a thiol-ene click chemical reaction.

A method of respectively dissolving dopamine and HPG using 1 M Tris-HCl pH 8.5 buffer, and then sequentially coating therewith and drying for 24 hours was applied. A buffer solution containing 2 mg/ml of dopamine was prepared, the substrate was immersed therein for 24 hours, and then thoroughly washed with the buffer solution. After drying, the substrate was coated in a way of immersing it in a buffer solution in which 2 mg/ml of HS-HPG was dissolved for 24 hours, and then thoroughly washed with the buffer solution (FIG. 8a).

As a result of measuring contact angles, contact angles of 16°, 24°, 37° and 55° were achieved (FIG. 8b).

2-2. Dip-coating/spray coating in methanol solvent environment

Coating was performed by methods different from the dopamine and HPG polymer coating method described in 2-1 above. Specifically, after changing the solvent from the 1.0 M Tris-HCl pH 8.5 buffer used in 2-1 to methanol, methods of dip-coating and spray coating, which are different from the dopamine coating and HPG polymer coating method, were respectively performed (FIG. 9a).

As the dip-coating method used for dopamine coating, a method of immersing a substrate in a 40 mM dopamine solution added with an oxidant for 5 minutes and then drying was used. Specifically, dopamine was dissolved in methanol to prepare a dopamine solution, the TPU substrate was immersed in the prepared solution, and coating was performed by putting an oxidant ($NaIO_4$). Thereafter, as the spray coating method used for HPG coating, a method of spraying the HPG solution on the substrate using a sprayer and then drying was performed.

As a result, the contact angle after dopamine dip-coating was 61°-76°, and after spray coating with HPG, the contact angle was about 39° (FIG. 9b).

2-3. Methacrylated HPG coating

In order to fix more stably on the substrate, a crosslinking agent was used to induce HPG coating by crosslinking on the substrate.

As a reducing agent, a PETMP(pentaerythritol tetra(3-mercaptopropionate)) crosslinking agent having four thiol groups was used.

Methacrylated polymer coating at a high concentration of 100 mg/ml was performed for 5 seconds using methanol as a solvent, and then crosslinking was formed using a reducing agent. In the present invention, it was attempted to find conditions under which the lowest contact angle was formed by varying the concentration of the PETMP reducing agent. As a result, a contact angle of about 20° was achieved before washing under 25 mM PETMP condition. The contact angle after washing was measured to be about 47° (FIG. 10b).

Then, it was tried to find optimal conditions by varying the number and time of immersion in a PETMP 25 mM reducing agent solution dissolved in ethanol or cyclopentanone. Considering whether the pre-coated polymer would be washed away during crosslinking, the coating solvent was set to ethanol, which dissolves the polymer, and cyclopentanone, which does not dissolve the polymer. When the number of immersion was set to once (for 1 second), the contact angle of cyclopentanone (30°) was observed to be lower than that of ethanol (45°). Additional experiments were conducted under various conditions using cyclopentanone as a solvent. Observation was performed by differently setting the number of immersions to 5 times and the immersion time to 10 minutes, but the angles were confirmed to be 63° and 66°, respectively (FIG. 11b).

Methods and results of "2-1. Oxidation polymerization coating," "2-2. Coating in methanol solvent environment" and "2-3 Methacrylated HPG Coating" were collectively summarized in Table 1 below.

[TABLE 1]

| | Example 2-1 | Example 2-2 | Example 2-3 |
|---|---|---|---|
| Coating strategy | Coating using a thiol-ene click chemical reaction | Coating using high concentration | Coating using a crosslinking reaction between methacryl group and reducing agent |
| Coating method | HPG dip-coating after dopamine dip-coating on a polymer substrate (FIG. 8a) | HPG spray coating after dopamine dip-coating on a polymer substrate (FIG. 9a) | Dip-coating in a PETMP reducing agent solution after AHPG alone coating on a polymer substrate |
| Solvent and additive | Tris-HCl pH 8.5 buffer | Methanol | Cyclopentanone / PETMP |
| Contactangle after coating | About 15°-55° | About 30° | 30° |
| Evaluati on | Excellent | Very excellent | Very excellent |

## Claims

1. A composition for surface treatment of a polymer material comprising a hyperbranched polyglycerol comprising a glycidol-derived repeating unit and an unsubstituted or substituted acrylate group at least at one end thereof.

2. The composition for surface treatment of a polymer material according to claim 1, wherein the hyperbranched polyglycerol has a number average molecular weight of 500 to 500,000 g/mol.

3. The composition for surface treatment of a polymer material according to claim 1, wherein the hyperbranched polyglycerol is subjected to ring-opening polymerization of glycidol using tritylated triethylene glycol as an initiator.

4. The composition for surface treatment of a polymer material according to claim 1, wherein the substituted acrylate is an acrylate in which carbon No. 2 is substituted with C1 to C6 straight or branched chain alkyl.

5. The composition for surface treatment of a polymer material according to claim 1, wherein the hyperbranched polyglycerol is a hyperbranched polyglycerol in which at least some of its ends are methacrylated.

6. The composition for surface treatment of a polymer material according to claim 5, wherein the methacrylated hyperbranched polyglycerol is prepared by reacting the hyperbranched polyglycerol with glycidyl methacrylate.

7. The composition for surface treatment of a polymer material according to claim 1, wherein the polymer material includes at least one selected from a group consisting of thermoplastic polyurethane (TPU), polycarbonate (PC), polydimethylsiloxane (PDMS), polyvinyl chloride (PVC), polyethylene glycol (PEG), silicone, Teflon (PTFE), polystyrene, nylon, polyethylene terephthalate (PET), polyacrylate, polypropylene (PP), polyethylene (PE), polyetheretherketone (PEEK), polysulfone (PS), phenol resin, epoxy resin, polyglycolide (PGA), polylactide (PLLA), polycaprolactone (PCL), poly(lactic-co-glycolic acid) (PLGA), poly(ε-caprolactone-co-lactide) (PLCL), polydioxanone (PDO), polytrimethylene carbonate (PTMC), polyanhydride, polyorthoester, polyphosphagen, hyaluronic acid, alginic acid, chitosan, collagen, gelatin and polyamino acid.

8. A kit for surface treatment of a polymer material comprising: the composition according to any one of claims 1 to 7; and a crosslinking agent solution.

9. The kit for surface treatment of a polymer material according to claim 8, wherein the crosslinking agent solution is prepared by dissolving a crosslinking agent in C1 to C6 alcohol, C3 to C6 cycloalkanone, or C3 to C6 oxacycloalkane.

10. The kit for surface treatment of a polymer material according to claim 8, wherein the crosslinking agent is PETMP(pentaerythritol tetra(3-mercaptopropionate), pentaerythritol tetrakis(3-mercaptopropionate)).

11. An in-vivo implantation material comprising a polymer material subjected to surface treatment with the composition according to any one of claims 1 to 7.

12. The in-vivo implantation material according to claim 11, wherein the in-vivo implantation material is a continuous blood glucose monitoring sensor.

13. A method for surface treatment of a polymer material comprising treating at least a portion of a surface of the polymer material with the composition according to any one of claims 1 to 6 and a crosslinking agent.

14. The method for surface treatment of a polymer material according to claim 13, wherein the step of treating is performed by immersing the polymer material in the composition, and then immersing in the crosslinking agent.

15. The method for surface treatment of a polymer material according to claim 13, wherein the crosslinking agent is PETMP(pentaerythritol tetra(3-mercaptopropionate), pentaerythritol tetrakis(3-mercaptopropionate)).

16. The method for surface treatment of a polymer material according to claim 13, wherein the crosslinking agent is dissolved in C1 to C6 alcohol, C3 to C6 cycloalkanone, or C3 to C6 oxacycloalkane.

17. The method for surface treatment of a polymer material according to claim 13, wherein a concentration of the crosslinking agent is 10 mM to 30 mM.

18. The method for surface treatment of a polymer material according to claim 13, wherein the polymer material includes at least one selected from the group consisting of thermoplastic polyurethane (TPU), polycarbonate (PC), polydimethylsiloxane (PDMS), polyvinyl chloride (PVC), polyethylene glycol (PEG), silicone, Teflon (PTFE), polystyrene, nylon, polyethylene terephthalate (PET), polyacrylate, polypropylene (PP), polyethylene (PE), polyetheretherketone (PEEK), polysulfone (PS), phenol resin, epoxy resin, polyglycolide (PGA), polylactide (PLLA), polycaprolactone (PCL), poly(lactic-co-glycolic acid) (PLGA), poly(ε-caprolactone-co-lactide) (PLCL), polydioxanone (PDO), polytrimethylene carbonate (PTMC), polyanhydride, polyorthoester, polyphosphagen, hyaluronic acid, alginic acid, chitosan, collagen, gelatin and polyamino acid.

[FIG. 1]

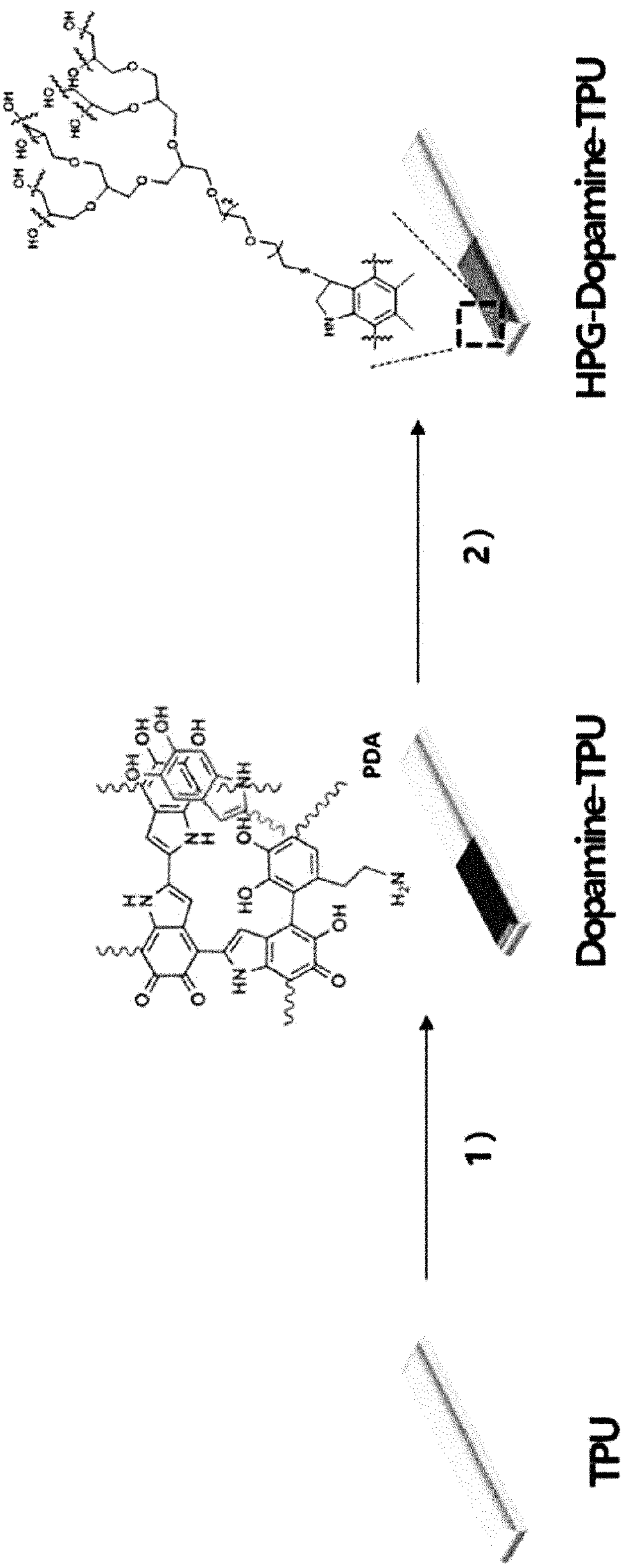

[FIG. 2]

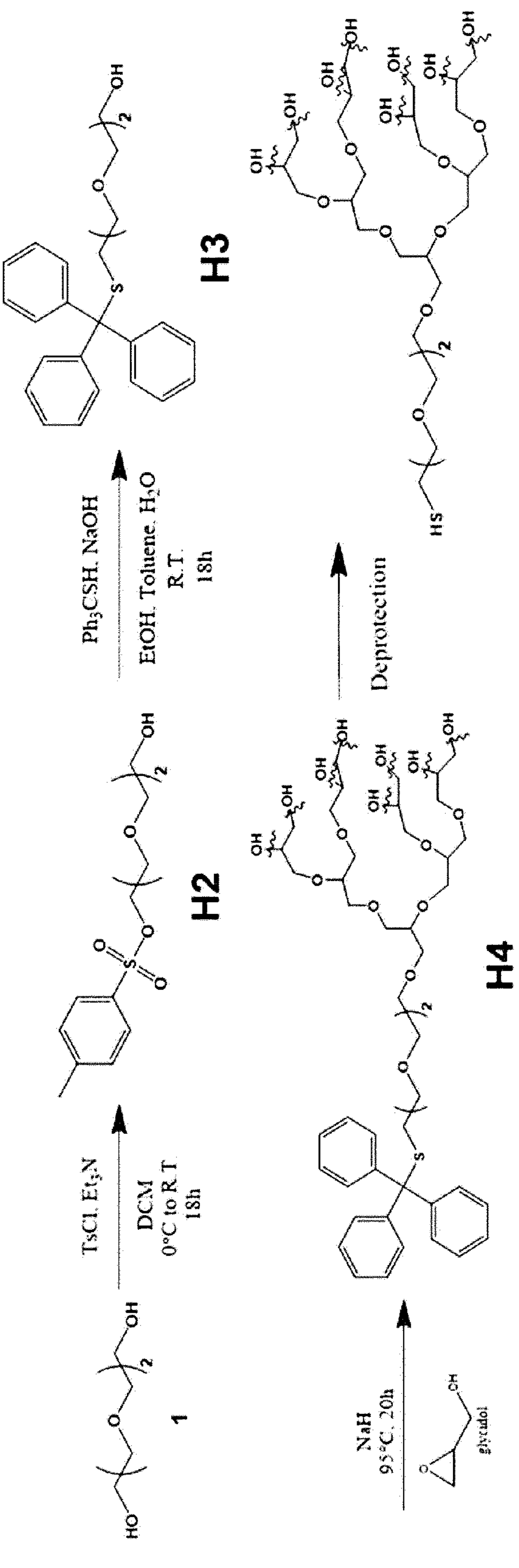


1

H2

H3

H4

[FIG. 3]

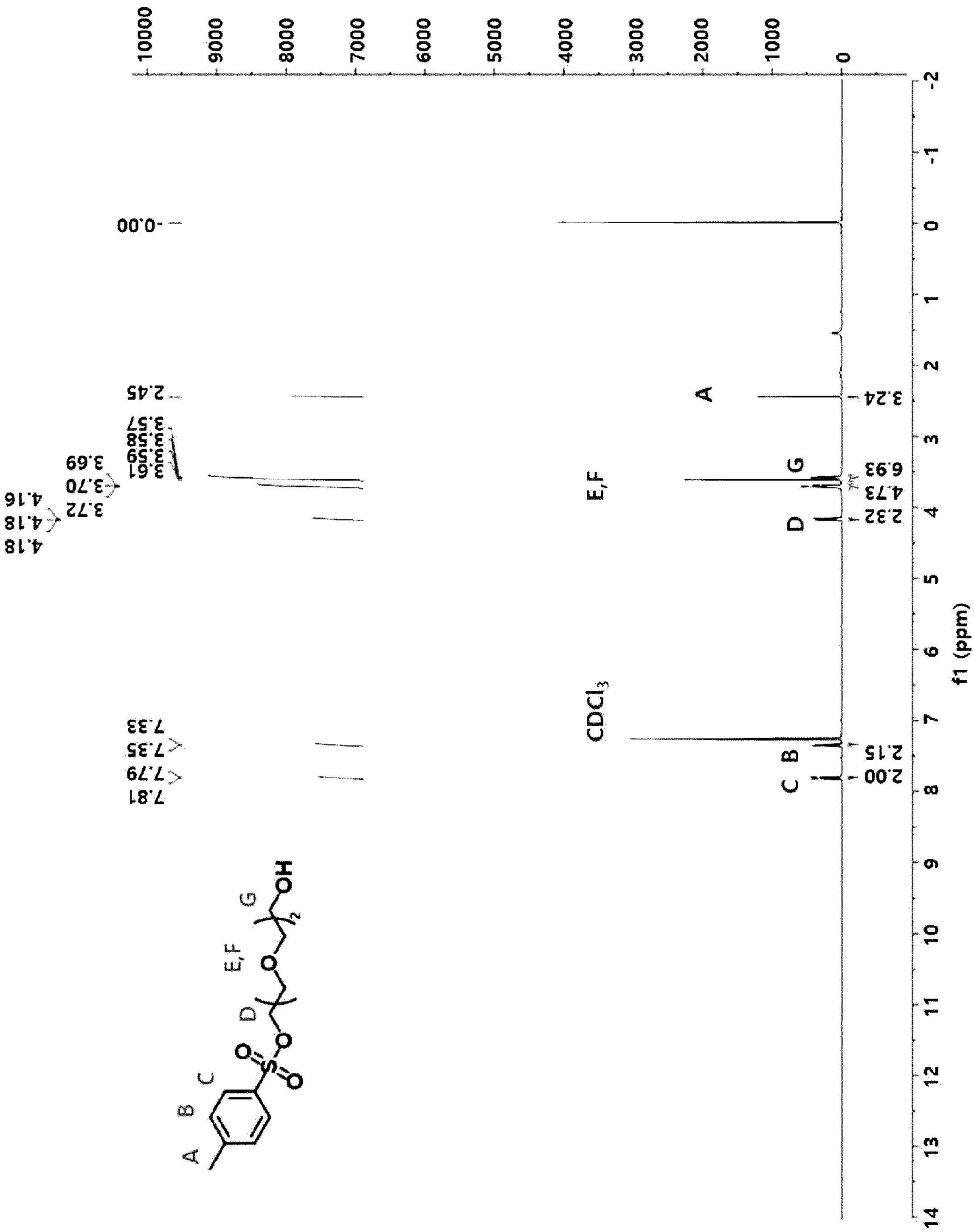

[FIG. 4]

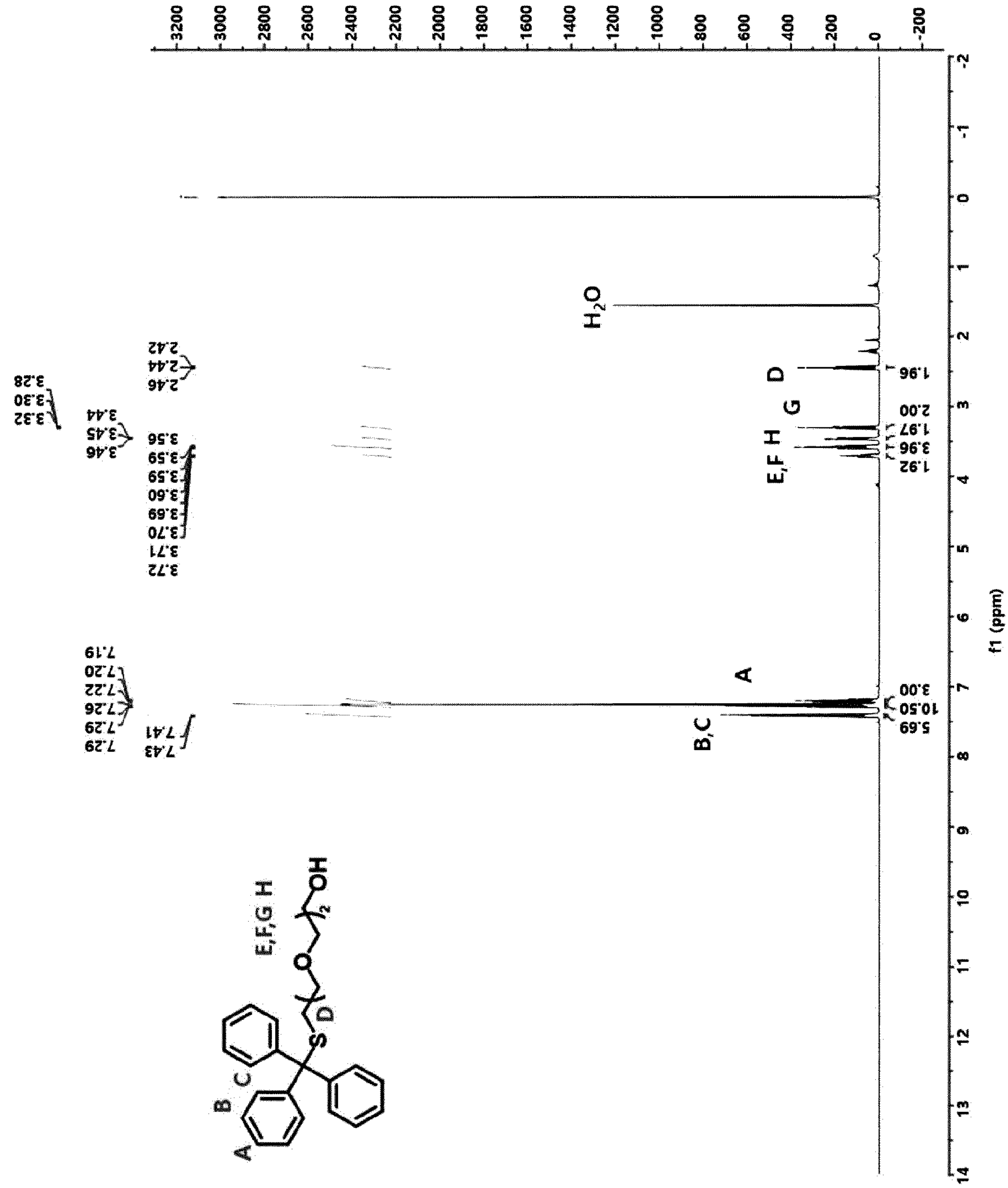

[FIG. 5]

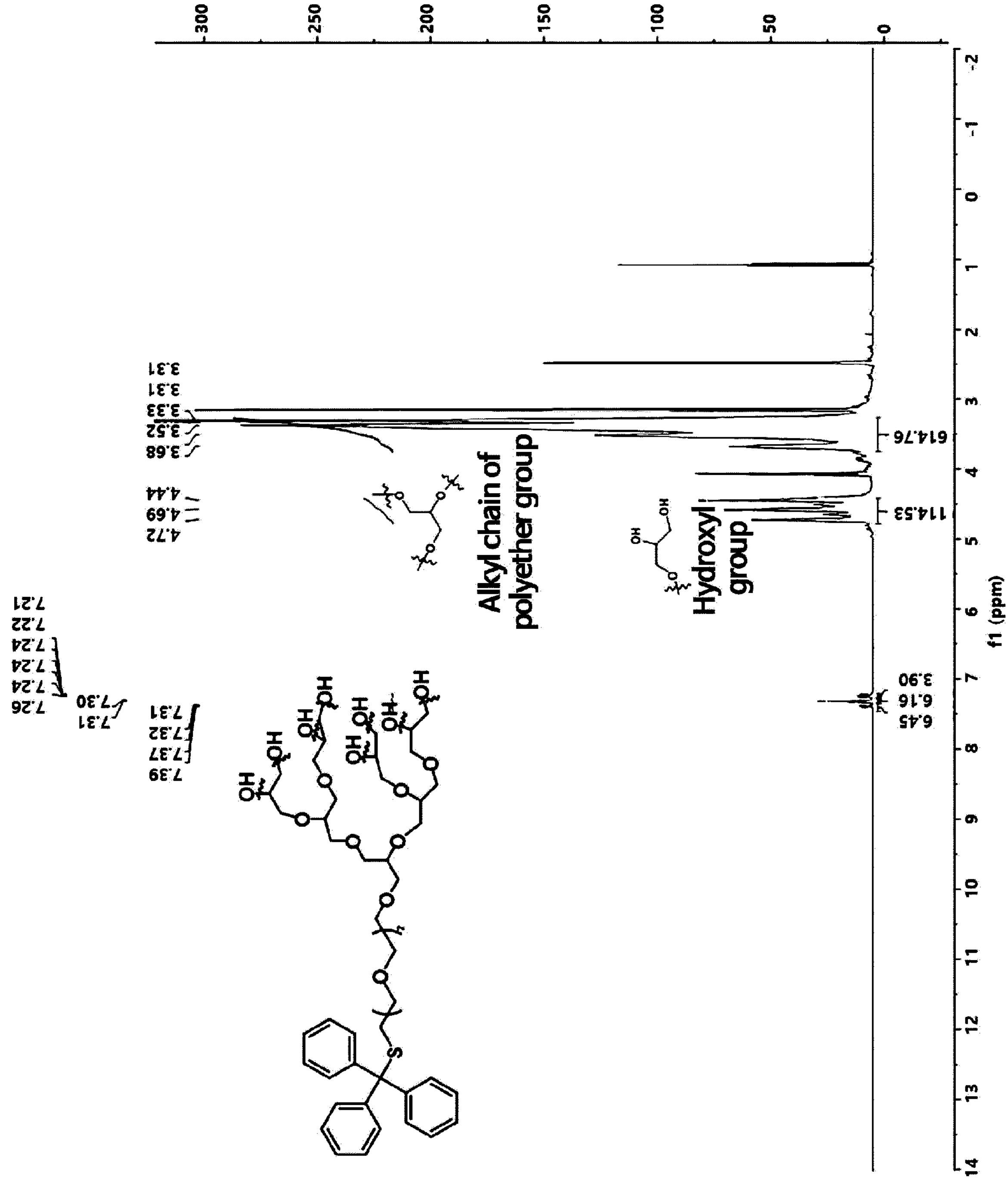
Alkyl chain of polyether group
Hydroxyl group
614.76
114.53
6.45
6.16
3.90
f1 (ppm)

[FIG. 6]

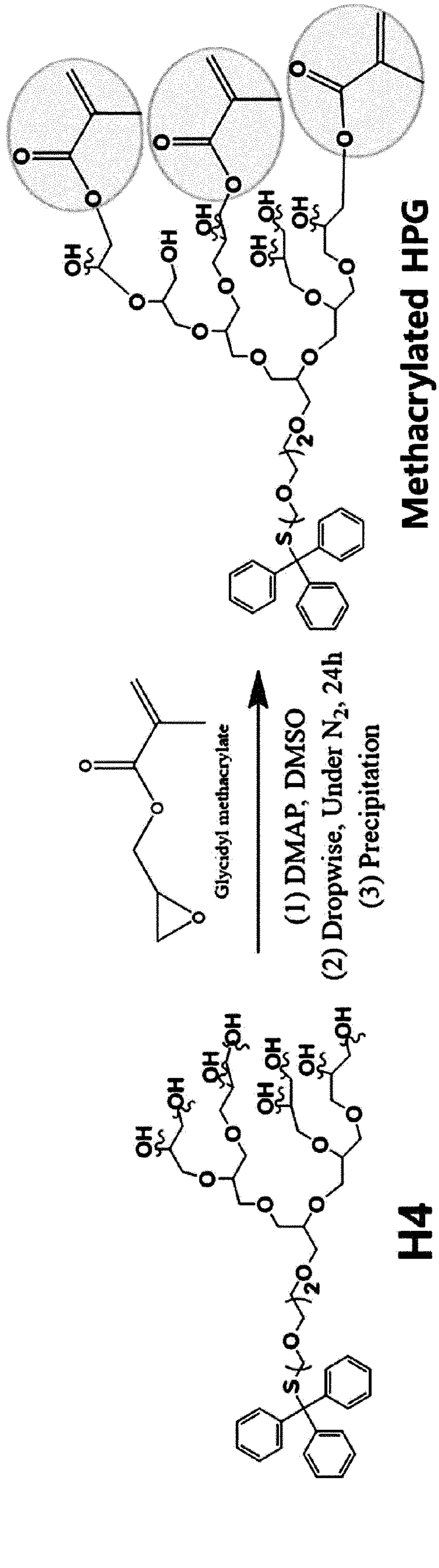
H4
Glycidyl methacrylate
(1) DMAP, DMSO
(2) Dropwise, Under $N_2$, 24h
(3) Precipitation
Methacrylated HPG

[FIG. 7]

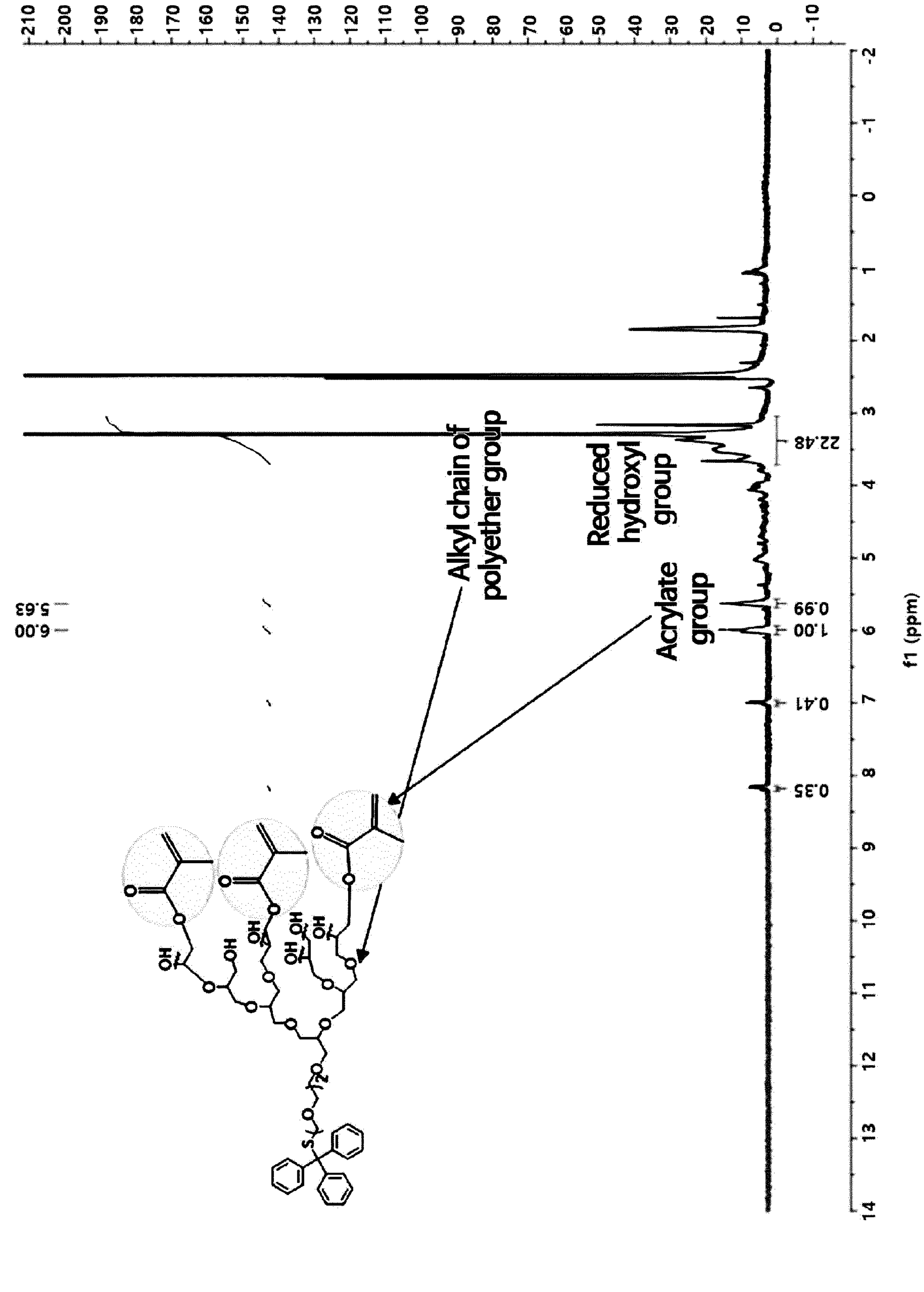
Alkyl chain of polyether group
Reduced hydroxyl group
Acrylate group
f1 (ppm)
22.48
0.99
1.00
0.41
0.35
5.63
6.00

[FIG. 8]

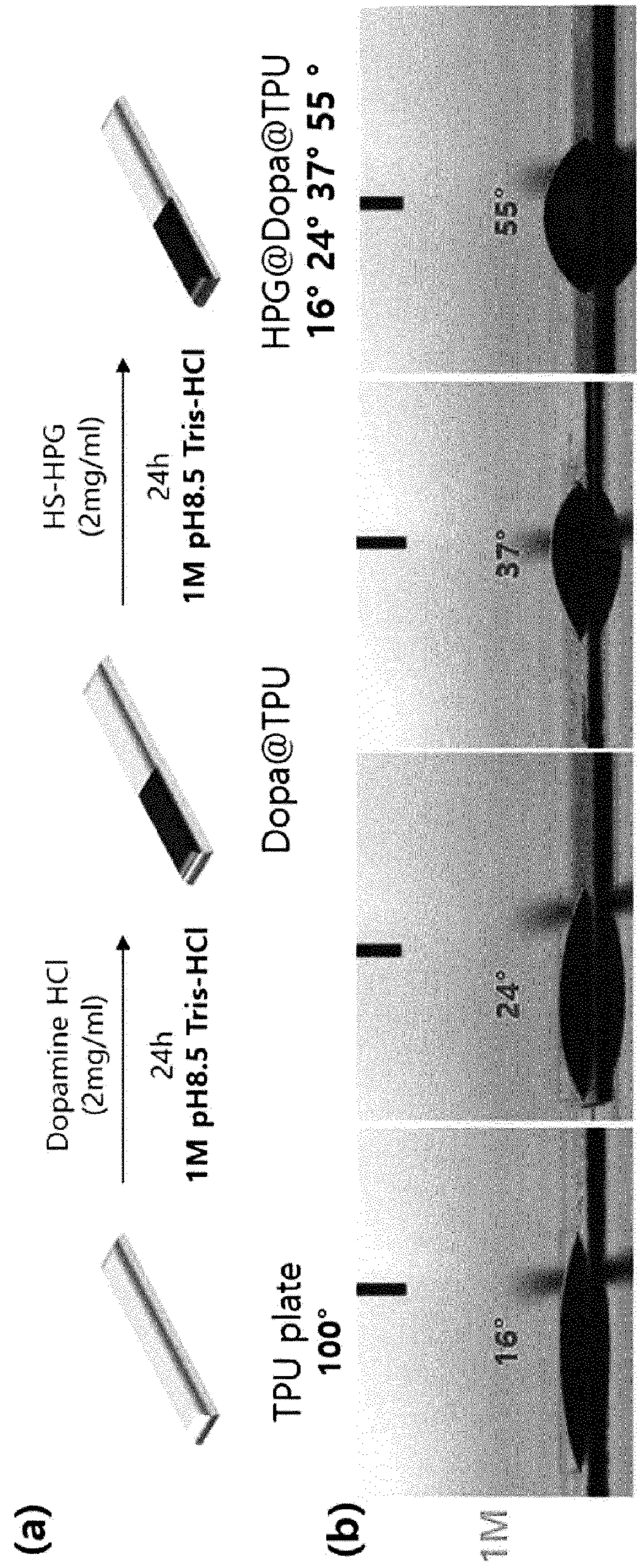
(a)
TPU plate
100°
Dopamine HCl
(2mg/ml)
24h
1M pH8.5 Tris-HCl
Dopa@TPU
HS-HPG
(2mg/ml)
24h
1M pH8.5 Tris-HCl
HPG@Dopa@TPU
16° 24° 37° 55 °
(b)
1M
16°
24°
37°
55°

[FIG. 9]

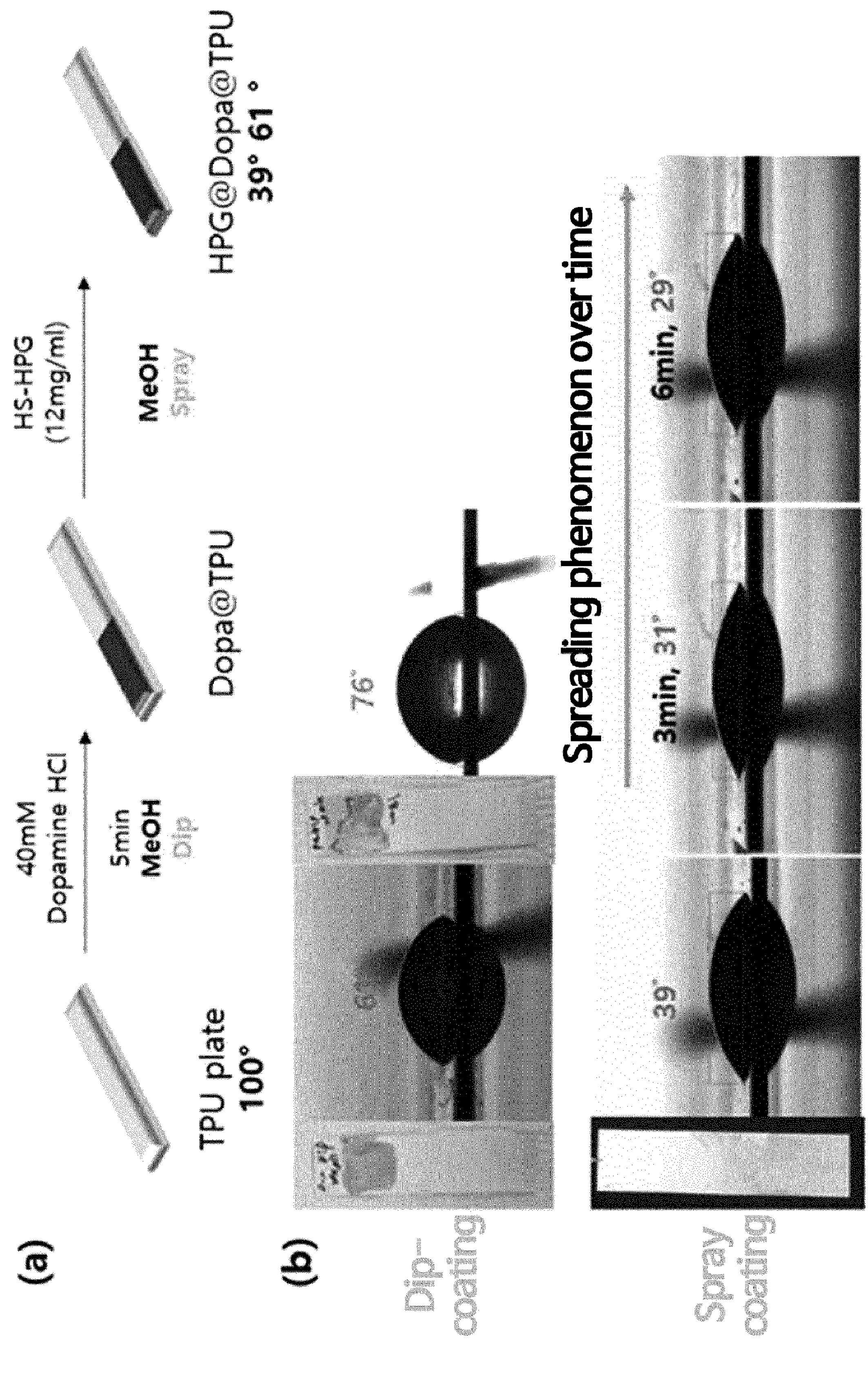
(a)
TPU plate
100°
40mM
Dopamine HCl
5min
MeOH
Dip
Dopa@TPU
HS-HPG
(12mg/ml)
MeOH
Spray
HPG@Dopa@TPU
39° 61 °
(b)
Dip-
coating
76°
Spray
coating
Spreading phenomenon over time
39°
3min, 31°
6min, 29°

[FIG. 10]

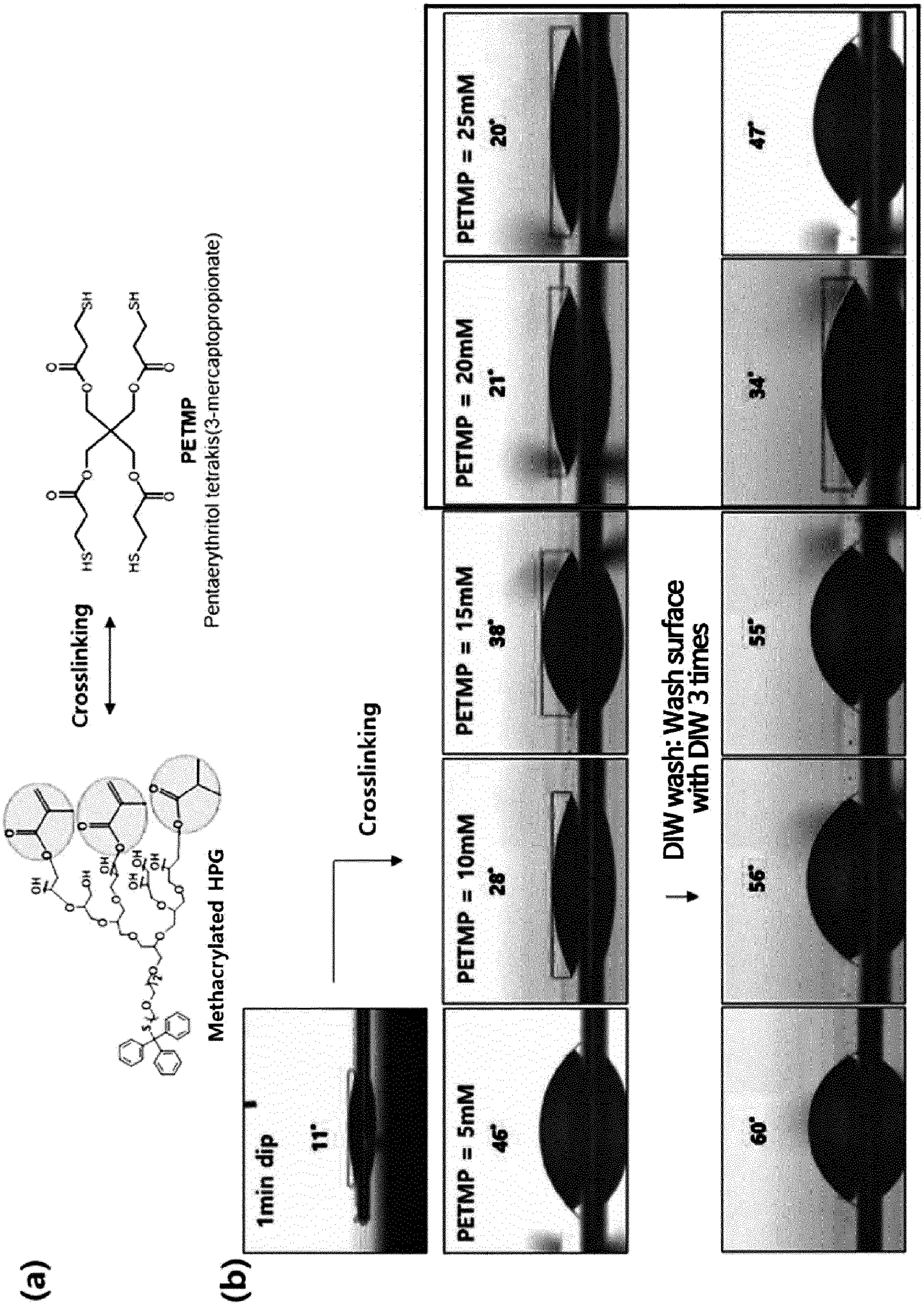
(a)
Methacrylated HPG
Crosslinking
PETMP
Pentaerythritol tetrakis(3-mercaptopropionate)
(b)
1min dip
11°
Crosslinking
PETMP = 5mM
46°
PETMP = 10mM
28°
PETMP = 15mM
38°
PETMP = 20mM
21°
PETMP = 25mM
20°
DIW wash: Wash surface with DIW 3 times
60°
56°
55°
34°
47°

[FIG. 11]

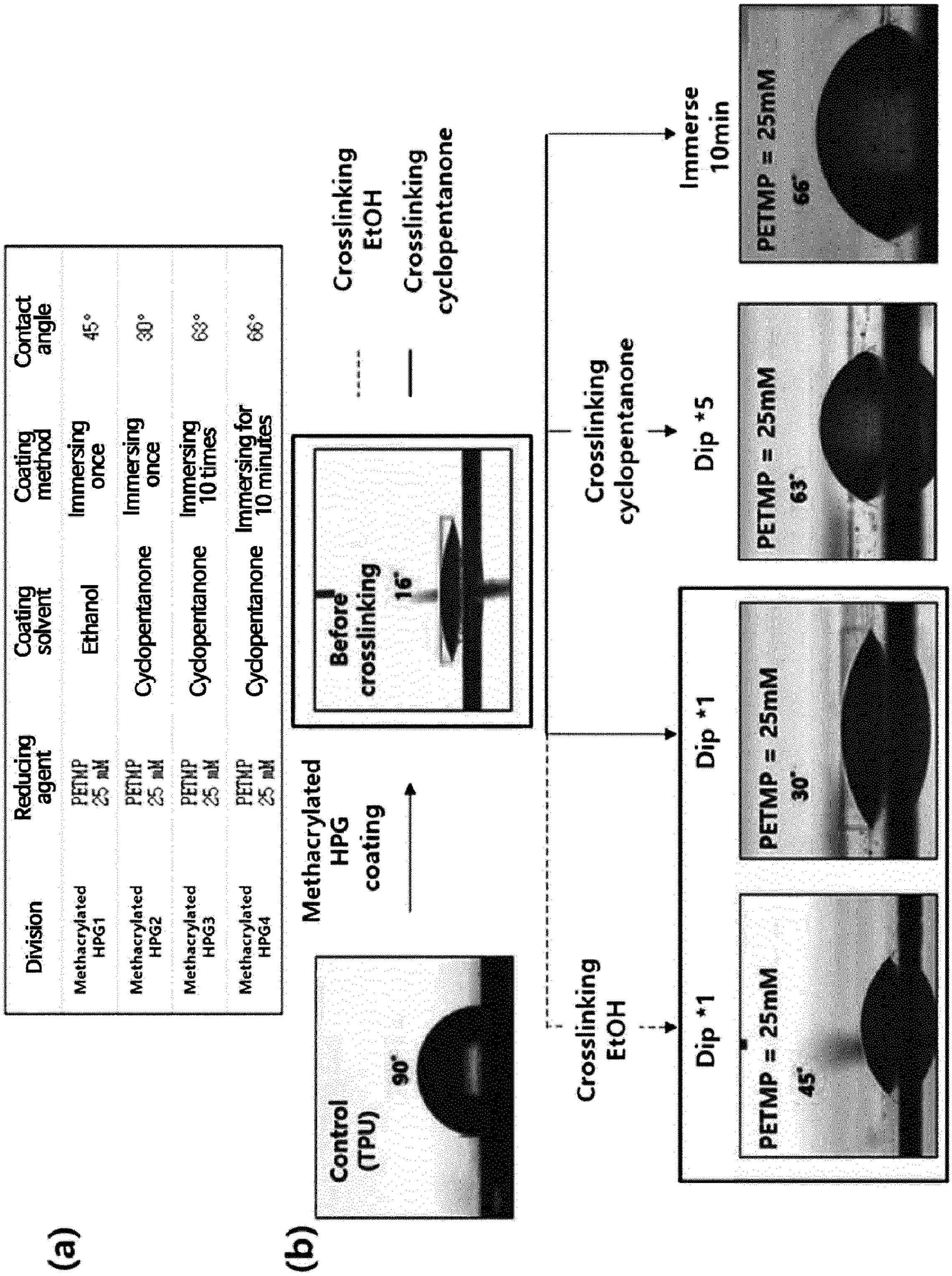


| Division | Reducing agent | Coating solvent | Coating method | Contact angle |
|---|---|---|---|---|
| Methacrylated HPG1 | PETMP 25 mM | Ethanol | Immersing once | 45° |
| Methacrylated HPG2 | PETMP 25 mM | Cyclopentanone | Immersing once | 30° |
| Methacrylated HPG3 | PETMP 25 mM | Cyclopentanone | Immersing 10 times | 63° |
| Methacrylated HPG4 | PETMP 25 mM | Cyclopentanone | Immersing for 10 minutes | 66° |

**INTERNATIONAL SEARCH REPORT**

International application No. **PCT/KR2022/021424**

**A. CLASSIFICATION OF SUBJECT MATTER**

**C08J 7/06**(2006.01)i; **A61L 27/34**(2006.01)i

According to International Patent Classification (IPC) or to both national classification and IPC

**B. FIELDS SEARCHED**

Minimum documentation searched (classification system followed by classification symbols)

C08J 7/06(2006.01); A01N 31/02(2006.01); A61L 29/06(2006.01); A61L 33/06(2006.01); C08G 65/26(2006.01); C08G 65/332(2006.01); C08G 65/34(2006.01); C08G 83/00(2006.01); C08K 5/1515(2006.01); C09D 11/033(2014.01); C09D 11/101(2014.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 고분자 재료(polymer material), 표면 처리(surface treatment), 글리시돌(glycidol), 하이퍼브랜치 폴리글리세롤(hyperbranch polyglycerol), 아크릴레이트(acrylate), 가교제(crosslinking agent)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | KR 10-2010-0080905 A (BASF SE) 13 July 2010 (2010-07-13)<br>See paragraphs [0017], [0020], [0045]-[0049], [0068]-[0079], [0141] and [0158]-[0171]. | 1,2,7-9,11-14,16-18<br>4-6<br>3,10,15 |
| Y | KR 10-1738356 B1 (CHUNGBUK NATIONAL UNIVERSITY INDUSTRY-ACADEMIC COOPERATION FOUNDATION) 22 May 2017 (2017-05-22)<br>See paragraphs [0005]-[0024], [0026]-[0067] and [0069]-[0083]. | 4-6 |
| A | KR 10-2020-0109139 A (UNIST(ULSAN NATIONAL INSTITUTE OF SCIENCE AND TECHNOLOGY)) 22 September 2020 (2020-09-22)<br>See entire document. | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 April 2023** | **04 April 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2022/021424**

**C. DOCUMENTS CONSIDERED TO BE RELEVANT**

| Category* | Citation of document, with indication, where appropriate, of the relevant passages | Relevant to claim No. |
|---|---|---|
| A | JP 2019-509874 A (TEKCYTE PTY LTD.) 11 April 2019 (2019-04-11) See entire document. | 1-18 |
| A | US 2020-0239642 A1 (QUANTIFOIL MICRO TOOLS GMBH et al.) 30 July 2020 (2020-07-30) See entire document. | 1-18 |

Form PCT/ISA/210 (second sheet) (July 2022)

**INTERNATIONAL SEARCH REPORT**
**Information on patent family members**

International application No.
**PCT/KR2022/021424**

| Patent document cited in search report | | | Publication date (day/month/year) | Patent family member(s) | | | Publication date (day/month/year) |
|---|---|---|---|---|---|---|---|
| KR | 10-2010-0080905 | A | 13 July 2010 | BR | PI0816251 | A2 | 17 March 2015 |
| | | | | CN | 101883810 | A | 10 November 2010 |
| | | | | EP | 2190901 | A1 | 02 June 2010 |
| | | | | JP | 2010-539073 | A | 16 December 2010 |
| | | | | US | 2009-0068138 | A1 | 12 March 2009 |
| | | | | WO | 2009-033970 | A1 | 19 March 2009 |
| KR | 10-1738356 | B1 | 22 May 2017 | | None | | |
| KR | 10-2020-0109139 | A | 22 September 2020 | KR | 10-2188574 | B1 | 08 December 2020 |
| | | | | US | 2020-0291253 | A1 | 17 September 2020 |
| JP | 2019-509874 | A | 11 April 2019 | AU | 2017-233547 | A1 | 21 September 2017 |
| | | | | AU | 2017-233547 | A1 | 25 October 2018 |
| | | | | AU | 2017-233547 | B2 | 03 March 2022 |
| | | | | CA | 3017779 | A1 | 21 September 2017 |
| | | | | CN | 109069695 | A | 21 December 2018 |
| | | | | CN | 109069695 | B | 16 November 2021 |
| | | | | EP | 3429651 | A1 | 23 January 2019 |
| | | | | EP | 3429651 | B1 | 21 December 2022 |
| | | | | JP | 7043700 | B2 | 30 March 2022 |
| | | | | US | 10994059 | B2 | 04 May 2021 |
| | | | | US | 2019-0076585 | A1 | 14 March 2019 |
| | | | | WO | 2017-156592 | A1 | 21 September 2017 |
| US | 2020-0239642 | A1 | 30 July 2020 | EP | 3619733 | A1 | 11 March 2020 |
| | | | | US | 11578173 | B2 | 14 February 2023 |
| | | | | WO | 2018-202837 | A1 | 08 November 2018 |

Form PCT/ISA/210 (patent family annex) (July 2022)